(19) Europäisches Patentamt
European Patent Office
Office européen des brevets

(11) Veröffentlichungsnummer: **0 031 911**
**B1**

(12) # EUROPÄISCHE PATENTSCHRIFT

(45) Veröffentlichungstag der Patentschrift: **17.11.82**

(51) Int. Cl.³: **A 01 N 43/64**, C 07 D 249/08

(21) Anmeldenummer: **80107695.1**

(22) Anmeldetag: **06.12.80**

(54) **Substituierte Triazolylmethyl-tert.-butyl-ketone, Verfahren zu ihrer Herstellung und ihre Verwendung als Pflanzenschutzmittel sowie als Zwischenprodukte.**

(30) Priorität: **19.12.79 DE 2951164**

(43) Veröffentlichungstag der Anmeldung: **15.07.81 Patentblatt 81/28**

(45) Bekanntmachung des Hinweises auf die Patenterteilung: **17.11.82 Patentblatt 82/46**

(84) Benannte Vertragsstaaten: **AT BE CH DE FR GB IT LI NL SE**

(56) Entgegenhaltungen:
**EP-A-0 010 183**
**DE-A-2 613 167**
**DE-A-2 734 426**
**DE-A-2 737 489**
**DE-A-2 805 227**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Krämer, Wolfgang, Dr., Am Eckbusch 39/45, D-5600 Wuppertal 1 (DE)**
Erfinder: **Büchel, Karl Heinz, Prof. Dr., Dabringhausener Strasse 42, D-5093 Burscheid (DE)**
Erfinder: **Frohberger, Paul-Ernst, Dr., Willi-Baumeister-Strasse 5, D-5090 Leverkusen 1 (DE)**
Erfinder: **Brandes, Wilhelm, Dr., Eichendorffstrasse 3, D-5653 Leichlingen 1 (DE)**
Erfinder: **Lürssen, Klaus, Dr., August-Kierspel-Strasse 89, D-5060 Bergisch Gladbach 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

EP 0 031 911 B1

Substituierte Triazolylmethyl-tert.-butyl-ketone, Verfahren zu ihrer Herstellung und ihre Verwendung als Pflanzenschutzmittel sowie als Zwischenprodukte

Die vorliegende Erfindung betrifft neue substituierte Triazolylmethyl-tert.-butyl-ketone, ein Verfahren zu ihrer Herstellung sowie ihre Verwendung als Pflanzenschutzmittel und als Zwischenprodukte für die Synthese von weiteren Pflanzenschutzmitteln.

Aus der DE-OS 2 805 227 und der DE-OS 2 734 426 sind bereits zahlreiche Triazolylmethyl-tert.-butylketone mit pflanzenwuchsregulierenden und fungiziden Eigenschaften bekannt. Entsprechende Stoffe, die eine durch Halogen substituierte tert.-Butyl-Gruppe enthalten, wurden jedoch bisher noch nicht offenbart. Von den in der DE-OS 2 805 227 und der DE-OS 2 734 426 beschriebenen Stoffen läßt sich beispielsweise das 1-(4-Chlorphenyl)-4,4-dimethyl-2-(1,2,4-triazol-1-yl)-pentan-3-on als Fungizid verwenden. Bei niedrigen Aufwandmengen läßt die Wirksamkeit dieser Verbindung in manchen Fällen allerdings zu wünschen übrig.

Weiterhin ist bereits bekanntgeworden, daß bestimmte Triazolyl-keton-Derivate gute fungizide Wirksamkeit besitzen. So kann zum Beispiel das 1-(2,4-Dichlorphenoxy)-3,3-dimethyl-1-(1,2,4-triazol-1-yl)-butan-2-on zur Bekämpfung von Pilzen eingesetzt werden (vgl. DE-AS 2 201 063). Auch die Wirkung dieses Stoffes ist jedoch nicht immer ganz befriedigend.

Es wurden un die neuen substituierten Triazolylmethyl-tert.-butyl-ketone der allgemeinen Formel

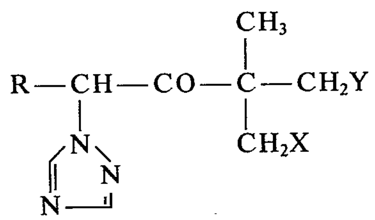

(I)

in welcher

R für Alkyl mit 1 bis 12 Kohlenstoffatomen, Alkenyl mit 2 bis 12 Kohlenstoffatomen, Alkinyl mit 2 bis 12 Kohlenstoffatomen, gegebenenfalls durch Alkyl mit 1 bis 4 Kohlenstoffatomen substituiertes Cycloalkyl mit 3 bis 7 Kohlenstoffatomen, gegebenenfalls durch Alkyl mit 1 bis 4 Kohlenstoffatomen substituiertes Cycloalkylalkyl mit 3 bis 7 Kohlenstoffatomen im Cycloalkylteil und 1 bis 4 Kohlenstoffatomen im Alkylteil sowie für Aralkyl mit 6 bis 10 Kohlenstoffatomen im Arylteil und 1 bis 4 Kohlenstoffatomen im Alkylteil steht, wobei der Arylteil substituiert sein kann durch Alkyl mit 1 bis 4 Kohlenstoffatomen, Alkoxy mit 1 bis 4 Kohlenstoffatomen, Alkylthio mit 1 bis 4 Kohlenstoffatomen, Halogen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Halogenalkylthio und 1 bis 4 Kohlenstoffatomen und 1 bis 5 gleichen oder verschiedenen Halogenatomen, Nitro, Cyano, Alkoxycarbonyl mit 1 bis 4 Kohlenstoffatomen im Alkoxyteil, Amino, Alkylamino mit 1 bis 4 Kohlenstoffatomen, Dialkylamino mit 1 bis 4 Kohlenstoffatomen in jedem Alkylteil, Phenylamino, gegebenenfalls durch Halogen oder Alkyl mit 1 oder 2 Kohlenstoffatomen substituiertes Phenoxy und/oder gegebenenfalls durch Halogen oder Alkyl mit 1 oder 2 Kohlenstoffatomen substituiertes Phenyl,

X für Wasserstoff steht und

Y für Fluor oder Chlor steht oder

X und Y gleich sind und für Fluor oder Chlor stehen,

sowie deren pflanzenverträgliche Säureadditions-Salze und Metallsalz-Komplexe gefunden.

Weiterhin wurde gefunden, daß man die substituierten Triazolylmethyl-tert.-butyl-ketone der Formel (I) sowie deren pflanzenverträgliche Säureadditions-Salze und Metallsalz-Komplexe erhält, wenn man Triazolylmethyl-tert.-butyl-ketone der Formel

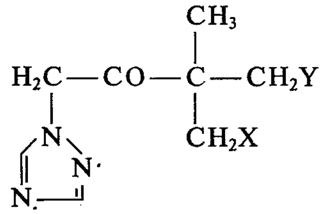

(II)

in welcher

X und Y die oben angegebene Bedeutung haben,

mit einem Alkylierungsmittel der allgemeinen Formel

R–Z (III)

in welcher

R die oben angegebene Bedeutung hat und
Z für eine elektronenanziehende Abgangsgruppierung steht,

in Gegenwart einer Base und in Gegenwart eines organischen Verdünnungsmittels, oder in einem wäßrig-organischen Zweiphasensystem in Gegenwart eines Phasentransferkatalysators, umsetzt und gegebenenfalls anschließend eine Säure oder ein Metallsalz addiert.

Die neuen substituierten Triazolylmethyl-tert.-butyl-ketone der Formel (I) sowie deren Säureadditions-Salze und Metallsalz-Komplexe weisen starke fungizide und pflanzenwachstumsregulierende Eigenschaften auf und sind daher als Pflanzenschutzmittel verwendbar.

Überraschenderweise zeigen die erfindungsgemäßen substituierten Triazolylmethyl-tert.-butyl-ketone eine bessere fungizide Wirkung als die aus dem Stand der Technik bekannten Triazolyl-keton-Derivate 1-(2,4-Dichlorphenoxy)-3,3-dimethyl-1-(1,2,4-triazol-1-yl)-butan-2-on und 1-(4-Chlorphenyl)-4,4-dimethyl-2-(1,2,4-triazol-1-yl)-pentan-3-on, die chemisch und wirkungsmäßig naheliegende Verbindungen sind.

Außerdem sind die neuen substituierten Triazolylmethyl-tert.-butyl-ketone interessante Zwischenprodukte zur Herstellung von weiteren Pflanzenschutz-Wirkstoffen.

Besonders bevorzugt sind diejenigen Verbindungen der Formel (I), in denen R für Methyl, Ethyl, n-Propyl, Isopropyl, n-Butyl, Isobutyl, sec.-Butyl, tert.-Butyl, Vinyl, Allyl, Butenyl, Propargyl, Butinyl, Cyclohexyl, Cyclohexylmethyl, sowie für Benzyl oder Naphthylmethyl steht, die gegebenenfalls einfach oder mehrfach, gleich oder verschieden substituiert sein können durch Methyl, Ethyl, Isopropyl, Methoxy, Methylthio, Fluor, Chlor, Trifluormethyl, Trifluormethoxy, Trifluormethylthio, Nitro, Cyano, Phenyl oder Phenoxy; und X und Y für die obengenannten Reste stehen.

Im einzelnen seien außer den bei den Herstellungsbeispielen genannten Verbindungen folgende Verbindungen der allgemeinen Formel (I) genannt:

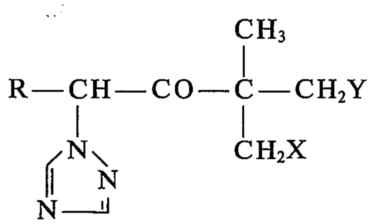

(I)

| X | Y | R |
|---|---|---|
| H | Cl | $CH_3$ |
| H | Cl | $C_2H_5$ |
| H | Cl | $i\text{-}C_3H_7$ |
| H | Cl | $C_4H_9$ |
| H | Cl | $-CH_2-CH=CH_2$ |
| H | Cl | $-CH_2-C\equiv CH$ |
| H | Cl | 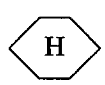 |
| H | Cl | 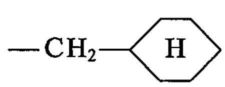 |
| H | Cl | 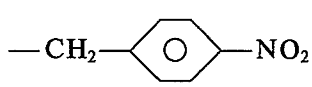 |

Fortsetzung

| X | Y | R |
|---|---|---|
| H | Cl | 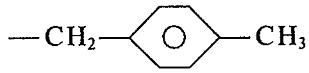 |
| H | Cl | 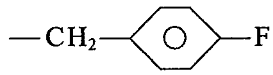 |
| H | Cl | 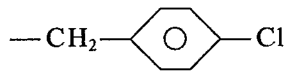 |
| H | Cl | 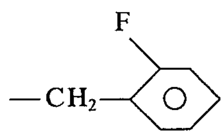 |
| H | Cl | 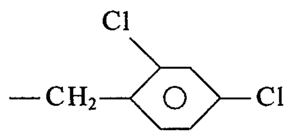 |
| H | Cl | 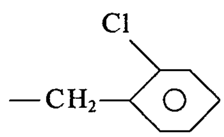 |
| H | Cl | 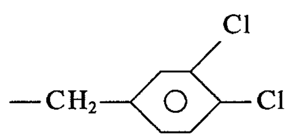 |
| H | Cl | 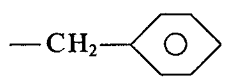 |
| H | Cl | 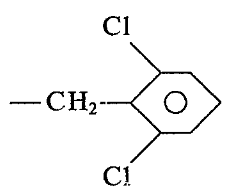 |
| H | Cl | 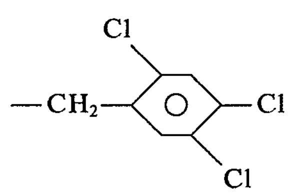 |
| H | F | 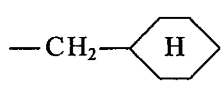 |
| H | F | 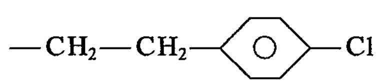 |
| H | F | 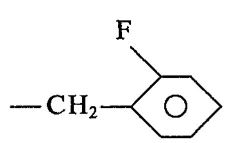 |

Fortsetzung

| X | Y | R |
|---|---|---|
| H | F | 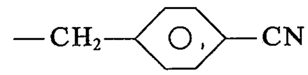 |
| H | F | 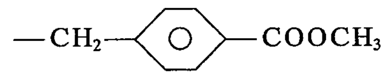 |
| Cl | Cl | $CH_3$ |
| Cl | Cl | $C_2H_5$ |
| Cl | Cl | i-$C_3H_7$ |
| Cl | Cl | $C_4H_9$ |
| Cl | Cl | $-CH_2-CH=CH_2$ |
| Cl | Cl | $-CH_2-C\equiv CH$ |
| Cl | Cl | 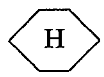 |
| Cl | Cl | 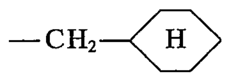 |
| Cl | Cl | 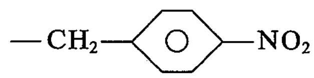 |
| Cl | Cl | 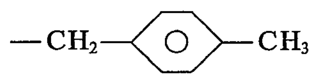 |
| Cl | Cl | 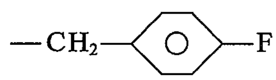 |
| Cl | Cl | 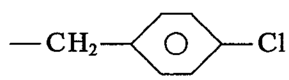 |
| Cl | Cl | 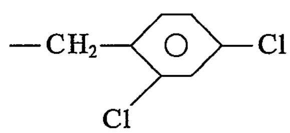 |
| Cl | Cl | 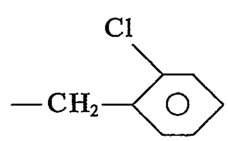 |
| Cl | Cl | 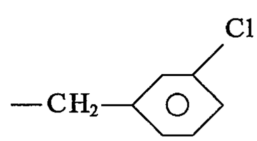 |
| Cl | Cl | 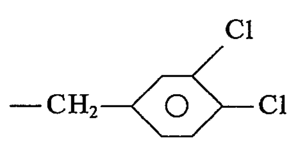 |

Fortsetzung

| X | Y | R |
|---|---|---|
| Cl | Cl | 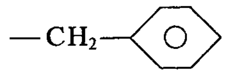 |
| Cl | Cl | 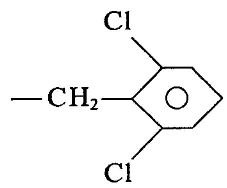 |
| Cl | Cl | 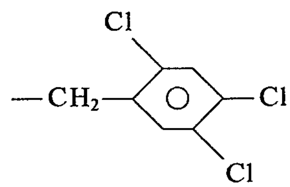 |
| F | F | $CH_3$ |
| F | F | $C_2H_5$ |
| F | F | i-$C_3H_7$ |
| F | F | $C_4H_9$ |
| F | F | $—CH_2—CH{=}CH_2$ |
| F | F | $—CH_2—C{\equiv}CH$ |
| F | F | 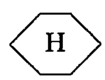 |
| F | F | 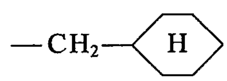 |
| F | F | 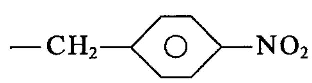 |
| F | F | 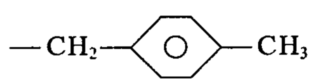 |
| F | F | 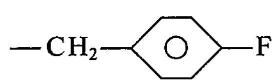 |
| F | F | 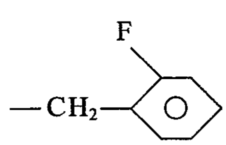 |
| F | F | 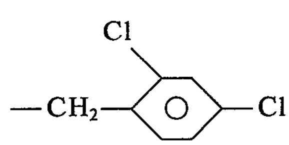 |

Fortsetzung

| X | Y | R |
|---|---|---|
| F | F | 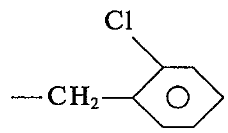 |
| F | F | 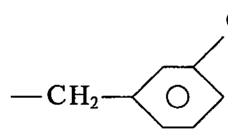 |
| F | F | 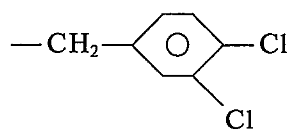 |
| F | F | 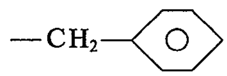 |
| F | F | 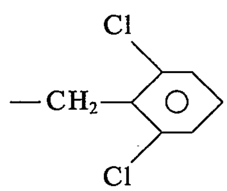 |
| F | F | 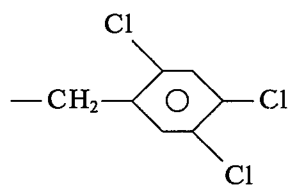 |

Verwendet man beispielsweise 1-Fluor-2,2-dimethyl-4-(1,2,4-triazol-1-yl)-butan-3-on und Methyljodid als Ausgangsstoffe, so kann der Reaktionsablauf durch das folgende Formelschema wiedergegeben werden:

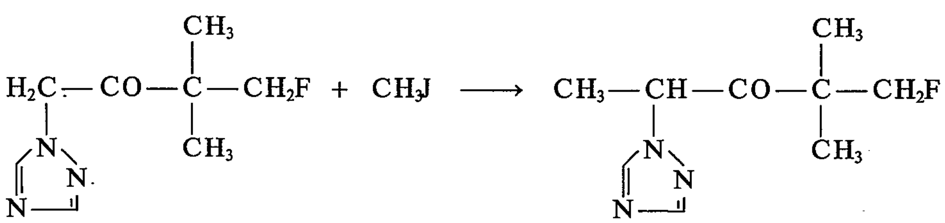

Die für die Durchführung des erfindungsgemäßen Verfahrens als Ausgangsstoffe benötigten Triazolylmethyl-tert.-butyl-ketone sind durch die Formel (II) allgemein definiert. In dieser Formel stehen X und Y für diejenigen Reste, die bereits im Zusammenhang mit der Beschreibung der erfindungsgemäßen Stoffe für X und Y genannt werden.

Die Triazolmethyl-tert.-butyl-ketone der Formel (II) sind bekannt oder lassen sich nach im Prinzip bekannten Verfahren herstellen (vgl. DE-OS 2 820 361). Man erhält Verbindungen der Formel (II), indem man Halogenketone der Formel

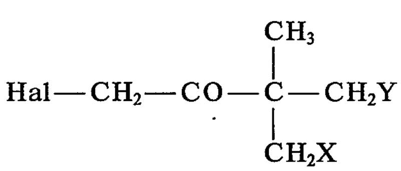

(IV)

in welcher

X und Y die oben angegebene Bedeutung haben und
Hal für Chlor oder Brom steht,

mit 1,2,4-Triazol in Gegenwart eines Verdünnungsmittels, wie beispielsweise Aceton, und in Gegenwart eines Säurebindemittels, wie beispielsweise Kaliumcarbonat, bei Temperaturen zwischen 20 und 150° C umsetzt.

Die Halogenketone der Formel (IV) werden erhalten, indem man Verbindungen der Formel

$$\begin{array}{c} \qquad\qquad\quad CH_3 \\ \qquad\qquad\quad | \\ CH_3—CO—C—CH_2Y \\ \qquad\qquad\quad | \\ \qquad\qquad\quad CH_2X \end{array} \qquad (V)$$

in welcher

X und Y die oben angegebene Bedeutung haben,

in einem inerten organischen Lösungsmittel bei Raumtemperatur mit Chlor oder Brom versetzt; oder z. B. mit üblichen Chlorierungsmitteln, wie Sulfurylchlorid, bei 20 bis 60° C umsetzt (vergleiche auch die Herstellungsbeispiele).

Als Beispiele für die Ausgangsstoffe der Formel (II) seien genannt:

1-Chlor-2,2-dimethyl-4-(1,2,4-triazol-1-yl)-butan-3-on
1-Fluor-2,2-dimethyl-4-(1,2,4-triazol-1-yl)-butan-3-on
2,2-Bis-chlormethyl-4-(1,2,4-triazol-1-yl)-butan-3-on
2,2-Bis-fluormethyl-4-(1,2,4-triazol-1-yl)-butan-3-on

Die außerdem für das erfindungsgemäße Verfahren als Ausgangsstoffe zu verwendenden Alkylierungsmittel sind durch die Formel (III) allgemein definiert. In dieser Formel steht R für diejenigen Reste, die bereits im Zusammenhang mit der Beschreibung der erfindungsgemäßen Stoffe der Formel (I) für diesen Substituenten genannt werden. Z steht für eine elektronenanziehende Abgangsgruppierung; vorzugsweise für Halogen, p-Methylphenylsulfonyloxy, die Gruppierung $-O-SO_2-OR''$ oder $\overset{\oplus}{N}R'_3$ und andere, wobei R′ vorzugsweise für Alkyl mit 1 bis 4 Kohlenstoffatomen steht.

Die Alkylierungsmittel der Formel (III) sind allgemein bekannte Verbindungen der organischen Chemie.

Für die erfindungsgemäße Umsetzung kommen als Verdünnungsmittel inerte organische Lösungsmittel in Frage. Hierzu gehören vorzugsweise aromatische Kohlenwasserstoffe, wie Benzol, Toluol oder Xylol; halogenierte Kohlenwasserstoffe, wie Methylenchlorid, Tetrachlorkohlenstoff, Chloroform oder Chlorbenzol; Ester, wie Essigester; Formamide, wie Dimethylformamid; sowie Dimethylsulfoxid.

Die erfindungsgemäße Umsetzung wird in Gegenwart einer Base durchgeführt. Hierbei können alle üblichen organischen und insbesondere anorganischen Basen eingesetzt werden, wie vorzugsweise Alkalihydroxide oder Alkalicarbonate, beispielsweise seien Natrium- und Kaliumhydroxid genannt.

Die Reaktionstemperaturen können bei der Durchführung des erfindungsgemäßen Verfahrens in einem größeren Bereich variiert werden. Im allgemeinen arbeitet man zwischen 0 und 100° C, vorzugsweise zwischen 20 und 100° C.

Bei der Durchführung des erfindungsgemäßen Verfahrens, setzt man vorzugsweise auf 1 Mol Triazolylmethyl-tert.-butyl-keton der Formel (II) 1 bis 1,2 Mol Alkylierungsmittel ein. Die Isolierung der Endprodukte der Formel (I) erfolgt in allgemein üblicher Art und Weise.

Die erfindungsgemäße Umsetzung kann auch in einem Zweiphasensystem, wie beispielsweise wäßrige Natron- oder Kalilauge/Toluol oder Methylenchlorid, gegebenenfalls unter Zusatz von 0,1 bis 1 Mol eines Phasen-Transfer-Katalysators, wie beispielsweise Ammonium- oder Phosphoniumverbindungen, beispielsweise seien Benzyldodecyl-dimethyl-ammoniumchlorid und Triethyl-benzyl-ammoniumchlorid genannt, durchgeführt werden.

Die erfindungsgemäß herstellbaren Verbindungen der Formel (I) können in Säureadditionssalze bzw. Metallsalz-Komplexe überführt werden.

Zur Herstellung von physiologisch verträglichen Säureadditionssalzen der Verbindungen der Formel (I) kommen vorzugsweise folgende Säuren in Frage: Die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure,

Zitronensäure, Salizylsäure, Sorbinsäure, Milchsäure, sowie Sulfonsäuren, wie z. B. p-Toluolsulfonsäure und 1,5-Naphthalindisulfonsäure. Die Säureadditions-Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe in Frage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z. B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z. B. durch Lösen des Metallsalzes in Alkohol, z. B. Ethanol, und Hinzufügen zur Verbindung der Formel (I). Man kann Metallsalz-Komplexe in bekannter Weise, z. B. durch Abfiltrieren, Isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflenzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten, wie Getreidemehltau (Erysiphe graminis) und die Streifenkrankheit der Gerste; von Erysiphe-Arten, wie den Erreger des Gurkenmehltaus (Erysiphe cichoracearum); von Fusicladium-Arten, wie den Erreger des Apfelschorfs (Fusicladium dendriticum); sowie von Reiskrankheiten, wie Pellicularia sasakii und Pyricularia oryzae, eingesetzt werden.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens (»Lagerns«) der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge, bezogen auf die Bodenfläche, erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch, daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der

Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z. B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z. B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle, ist aber auch in anderen Kulturen wie z. B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden (»Ausdünnung«), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z. B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid. Als feste Trägerstoffe kommen in Frage: z. B. natürliche Gesteinsmehle, wie Kaoline, Tonerden,

Talkum, Kreide, Quarz, Attapulgit, Montmorillonit oder Diatomeenerde und synthetische Gesteinsmehle, wie hochdisperse Kieselsäure, Aluminiumoxid und Silikate. Alf feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z. B. nichtionogene und anionische Emulgatoren, wie Polyoxyethylen-Fettsäureester, Polyoxyethylen-Fettalkoholether, z. B. Alkylarylpolyglycol-ether, Alkylsulfonate, Alkylsulfate, Arylsulfonate sowie Eiweißhydrolysate. Als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Allizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

Bei der Verwendung als Pflanzenwachstumsregulatoren können die Wirkstoffkonzentrationen in einem größeren Bereich variiert werden. Im allgemeinen verwendet man pro Hektar Bodenfläche 0,01 bis 50 kg, bevorzugt 0,05 bis 10 kg, an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

## Herstellungsbeispiele

### Beispiel 1

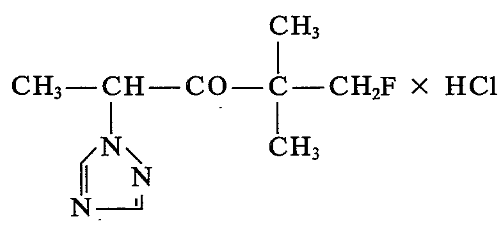

37,2 g (0,2 Mol) 1-Fluor-2,2-dimethyl-4-(1,2,4-triazol-1-yl)-butan-3-on werden in 200 ml Dimethylsulfoxid gelöst, 11,2 g (0,2 Mol) Kaliumhydroxid, in 24 ml Wasser gelöst, zugegeben und 28,4 g (0,2 Mol) Methyljodid unter Kühlung bei 20° C zugetropft. Man läßt 24 Stunden bei Raumtemperatur nachrühren, gibt das Reaktionsgemisch auf 1000 ml Wasser, extrahiert zweimal mit je 300 ml Methylenchlorid, wäscht die vereinigten organischen Phasen fünfmal mit je 100 ml Wasser, trocknet die organische Phase über Natriumsulfat, destilliert das Lösungsmittel ab, nimmt den Rückstand in 100 ml Aceton auf, filtriert und destilliert das Lösungsmittel der Mutterlauge ab. Der Rückstand wird in 150 ml Essigester aufgenommen und 14,4 g (0,2 Mol) Chlorwasserstoff eingeleitet. Danach läßt man auskristallisieren. Man erhält 33,8 g (72% der Theorie) 1-Fluor-2,2-dimethyl-4-(1,2,4-triazol-1-yl)-pentan-3-on-hydrochlorid vom Schmelzpunkt 142° C.

Herstellung des Ausgangsproduktes

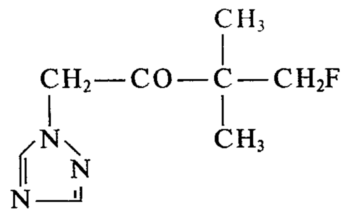

4,18 kg (35,4 Mol) 1-Fluor-2,2-dimethyl-butan-3-on werden in 30 l Methylenchlorid gelöst, 5,67 kg Brom bei 20°C innerhalb von 2 Stunden so zugetropft, daß laufend Entfärbung eintritt. Das Lösungsmittel wird im Wasserstrahlvakuum abdestilliert, nochmals 15 l Methylenchlorid zum Rückstand gegeben und erneut das Lösungsmittel im Wasserstrahlvakuum abdestilliert. Das rohe 1-Fluor-4-brom-2,2-dimethyl-butan-3-on (6,97 kg quantitative Ausbeute) wird zu 2,45 kg 1,2,4-Triazol, 4,89 kg Kaliumcarbonat in 21,4 l Aceton bei 30 bis 35°C unter Kühlung innerhalb von 2 Stunden zugetropft. Man läßt 15 Stunden bei Raumtemperatur nachrühren, filtriert vom Ungelösten ab und destilliert das Lösungsmittel im Wasserstrahlvakuum ab. Man erhält 6,12 kg (93% der Theorie) 1-Fluor-2,2-dimethyl-4-(1,2,4-trizaol-1-yl)-butan-3-on, das direkt weiter umgesetzt werden kann.

In entsprechender Weise werden die folgenden erfindungsgemäßen Verbindungen der allgemeinen Formel

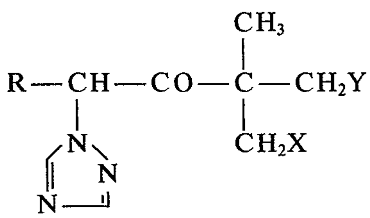

(I)

erhalten:

| Beispiel Nr. | R | X | Y | Schmelzpunkt (°C) bzw. Brechungsindex |
|---|---|---|---|---|
| 2 | 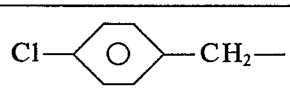 | H | F | 78–79 |
| 3 | 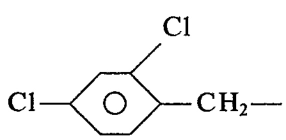 | H | F | 112–20 |
| 4 | 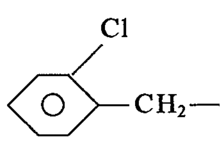 | H | F | 62–72 |
| 5 | 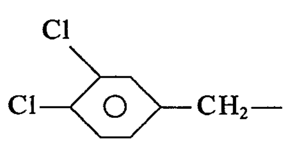 | H | F | 58–70 |
| 6 | 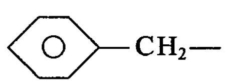 | H | F | 150 (Zers.) (× HCl) |
| 7 | 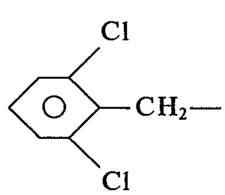 | H | F | 80–92 |

Fortsetzung

| Beispiel Nr. | R | X | Y | Schmelzpunkt (°C) bzw. Brechungsindex |
|---|---|---|---|---|
| 8 | 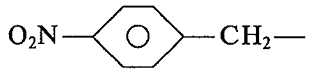 | H | F | 138–40 |
| 9 | i-$C_3H_7$— | H | F | 45 (× HCl) |
| 10 | 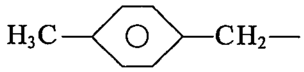 | H | F | 94 |
| 11 | $C_2H_5$ | H | F | $Kp_{0,05}$ 152 |
| 12 | 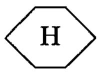 | H | F | 128 |
| 13 | $C_4H_9$ | H | F | $Kp_{0,05}$ 163 |
| 14 | $CH_2{=}CH{-}CH_2{-}$ | H | F | Öl |
| 15 | $CH{\equiv}C{-}CH_2{-}$ | H | F | 130 (Zers.) (× HCl) |
| 16 | 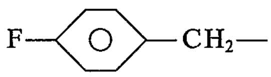 | H | F | 182 (Zers.) (× HCl) |
| 17 | 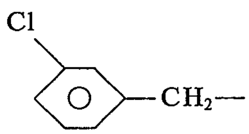 | H | F | 99 |
| 18 | 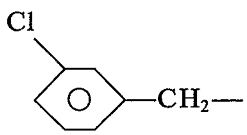 | H | Cl | 102 |
| 19 | 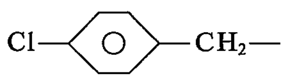 | F | F | 108 |
| 20 | 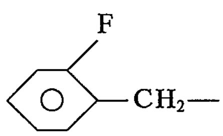 | Cl | Cl | Öl |
| 21 | 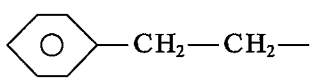 | H | F | Öl |
| 22 | 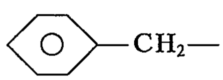 | F | F | Öl |
| 23 | 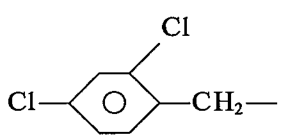 | F | F | 63–78 |
| 24 | 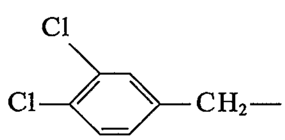 | F | F | 96–112 (× HCl) (Zersetz.) |

Fortsetzung

| Beispiel Nr. | R | X | Y | Schmelzpunkt (°C) bzw. Brechungsindex |
|---|---|---|---|---|
| 25 | 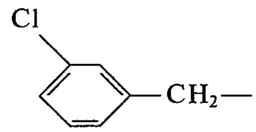 | H | Cl | 116–27 (× HCl) |
| 26 | 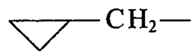 | H | F | 107 (× HCl) (Zersetz.) |
| 27 | 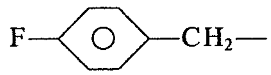 | H | Cl | 58–78 |
| 28 | 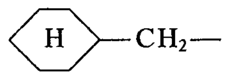 | H | F | 88–98 (× HCl) |
| 29 | 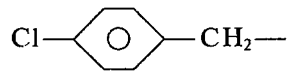 | H | Cl | 58–74 |
| 30 | $CH_2{=}CH{-}CH_2{-}CH_2{-}$ | H | F | Öl |
| 31 | 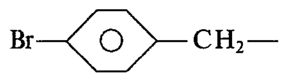 | H | F | 78 |
| 32 | 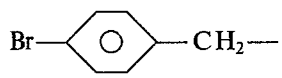 | H | Cl | 56 |
| 33 | 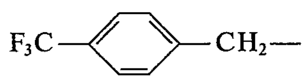 | H | F | 82 |
| 34 | 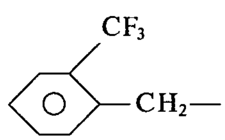 | H | F | 86 |
| 35 | 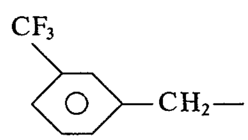 | H | F | 88 |
| 36 | 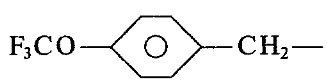 | H | F | 86–88 |
| 37 | 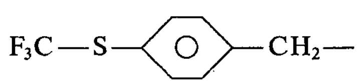 | H | F | 86–92 |
| 38 | 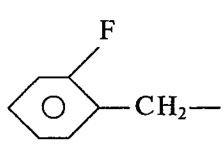 | H | F | 46–48 |
| 39 | 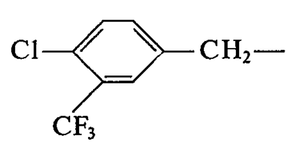 | H | F | 53–63 |

Fortsetzung

| Beispiel Nr. | R | X | Y | Schmelzpunkt (°C) bzw. Brechungsindex |
|---|---|---|---|---|
| 40 | 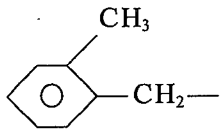 | H | F | 62–4 |
| 41 | 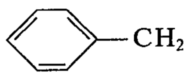 | Cl | Cl | Öl $n_D^{20}$ 1,5408 |
| 42 | 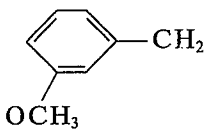 | H | F | 78–81 |
| 43 | 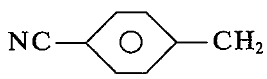 | H | F | 138–41 |

## Anwendungsbeispiele

Bei der Prüfung auf fungizide Wirksamkeit werden in den nachfolgenden Beispielen die nachstehend angegebenen Verbindungen als Vergleichssubstanzen eingesetzt:

A = 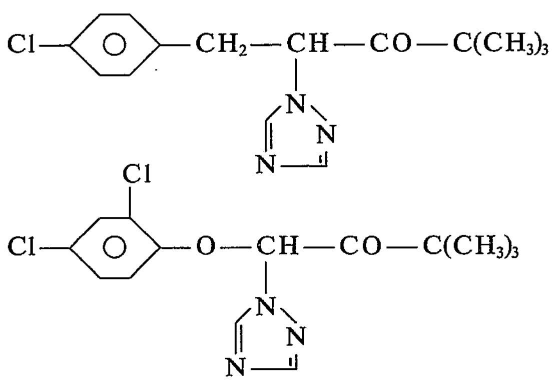

B =

## Beispiel A

Sproßbehandlungs-Test / Getreidemehltau / Protektiv
(blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator (Alkyl-aryl-polyglykolether) auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var. hordei.

Nach 6 Tagen Verweilzeit der Pflanzen bei einer Temperatur von 21–22° C und einer Luftfeuchtigkeit von 80–90% wertet man den Besatz der Pflanzen mit Mehltaupusteln aus. Der Befallsgrad wird in Prozent des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist um so wirksamer, je geringer der Mehltaubefall ist.

Bei diesem Test zeigen z. B. die folgenden Verbindungen eine sehr gute Wirkung, die derjenigen der aus dem Stand der Technik bekannten Verbindung (A) überlegen ist:

Verbindungen gemäß Herstellungsbeispielen 3 und 5.

Tabelle A

Sproßbehandlungs-Test/Getreidemehltau/Protektiv

| Wirkstoffe | Wirkstoff-konzentration in der Spritz-brühe in Gew.-% | Befall in % der unbehandelten Kontrolle |
|---|---|---|
| 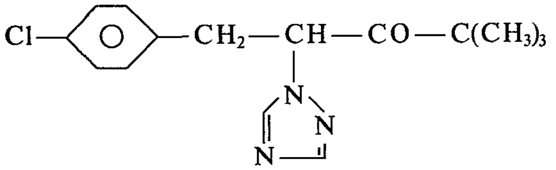 (A) | 0,001 | 72,5 |
| 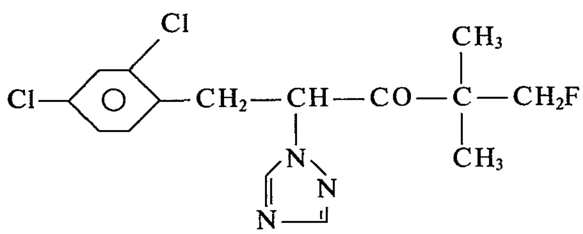 (3) | 0,001 | 0,0 |
| 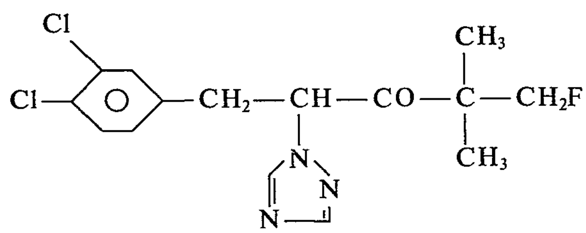 (5) | 0,001 | 0,0 |

Beispiel B

Fusicladium-Test (Apfel) / protektiv

Lösungsmittel: 4,7 Gewichtsteile Aceton
Emulgator: 0,3 Gewichtsteile Alkylarylpolyglykolether
Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20°C und einer relativen Luftfeuchtigkeit von 70% im Gewächshaus. Anschließend werden sie mit einer wäßrigen Konidiensuspension des Apfelschorferregers (Fusicladium dendriticum) inokuliert und 18 Stunden lang in einer Feuchtkammer bei 18 bis 20°C und 100% relativer Luftfeuchtigkeit inkubiert.

Die Pflanzen kommen dann erneut für 14 Tage ins Gewächshaus.

15 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

Bei diesem Test zeigen z. B. die folgenden Verbindungen eine sehr gute Wirkung, die derjenigen der aus dem Stand der Technik bekannten Verbindung (B) überlegen ist:

Verbindungen gemäß Herstellungsbeispiel 2.

Tabelle B

Fusicladium-Test(Apfel)/protektiv

| Wirkstoff | Befall in % bei einer Wirkstoff-konzentration von 0,00025 % |
|---|---|
| 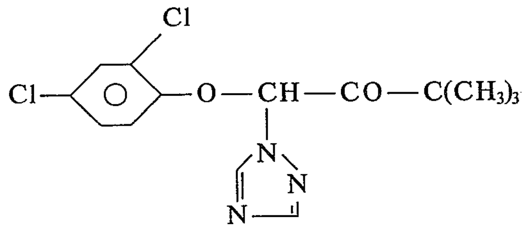 (B) | 46 |
| 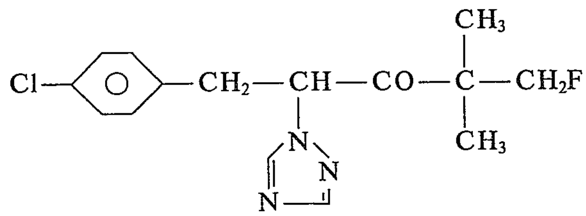 (2) | 39 |

Beispiel C

Wuchsbeeinflussung bei Zuckerrüben

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit der Wirkstoffzubereitung besprüht. Nach 14 Tagen wird der Zuwachs der Pflanzen gemessen und die Wuchsbeeinflussung in Prozent des Zuwachses der Kontrollpflanzen berechnet. Es bedeuten 0% Wuchsbeeinflussung ein Wachstum entsprechend dem der Kontrollpflanzen. Negative Werte kennzeichnen eine Wuchshemmung, positive Werte eine Wuchsförderung gegenüber den Kontrollpflanzen.

Die erfindungsgemäßen Wirkstoffe 2 und 7 zeigen in diesem Test gegenüber der Kontrolle eine starke Wuchsbeeinflussung.

Tabelle C

Wuchsbeeinflussung bei Zuckerrüben

| Wirkstoff | Konzentration in % | Wuchsbeeinflussung in % |
|---|---|---|
| 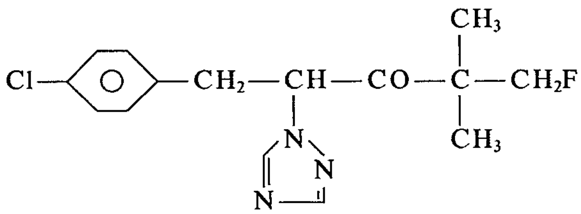 (2) | 0,05 | −45*) **) |
| 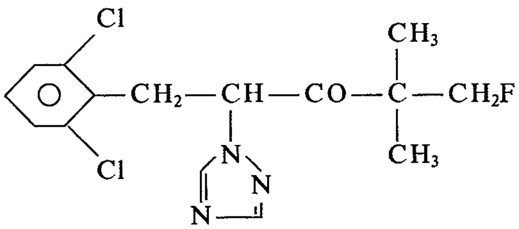 (7) | 0,05 | −50**) |
| **Kontrolle** | – | = 0 |

*) Dunkelgrüne Blätter.
**) Dicke Blätter.

Beispiel D

Wuchshemmung bei Sojabohnen

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Der erfindungsgemäße Wirkstoff 2 zeigt in diesem Test gegenüber der Kontrolle eine starke Wuchshemmung.

Tabelle D

Wuchshemmung bei Sojabohnen

| Wirkstoffe | Wirkstoff-konzentration in % | Wuchshemmung in % |
|---|---|---|
| 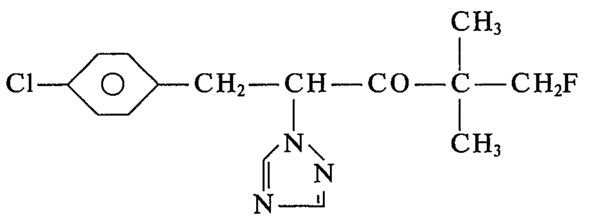 (2) | 0,05 | 70*) |
| Kontrolle | - | = 0 |

*) Dunkelgrüne Blattfarbe.

Beispiel E

Wuchshemmung bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit der angegebenen Menge Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Der erfindungsgemäße Wirkstoff 2 zeigt in diesem Test gegenüber der Kontrolle eine starke Wuchshemmung.

Tabelle E

Wuchshemmung bei Baumwolle

| Wirkstoff | Konzentration in % | Wuchshemmung in % |
|---|---|---|
| 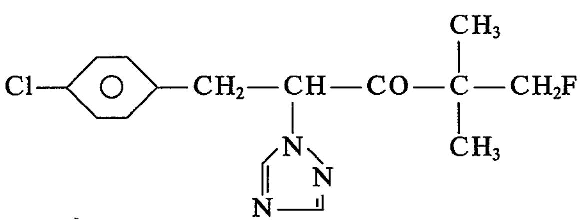 (2) | 0,05 | 50 |
| Kontrolle | – | = 0 |

Wie schon erwähnt, stellen die neuen substituierten Triazolylmethyl-tert.-butyl-ketone der Formel (I) auch interessante Zwischenprodukte dar. Sie lassen sich z. B. leicht in Triazolylmethyl-tert.-butyl-carbinole der Formel

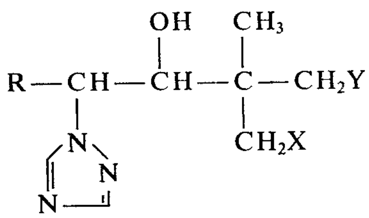 (VI)

in welcher

R, X und Y die oben angegebene Bedeutung haben,

überführen, indem man die Verbindungen der Formel (I) nach bekannten Methoden reduziert, wie z. B. durch Umsetzung mit komplexen Hydriden, wie insbesondere Natriumborhydrid, gegebenenfalls in Gegenwart eines polaren organischen Lösungsmittels, wie beispielsweise Alkohole, bei Temperaturen zwischen 0 und 30° C.

Die Verbindungen der Formel (VI) weisen starke fungizide Eigenschaften auf und sind daher als Pflanzenschutzmittel verwendbar.

Der nachfolgende Vergleichsversuch zeigt z. B. die überlegene Wirkung des 5-(4-Chlorphenyl)-2,2-dimethyl-1-fluor-4-(1,2,4-triazol-1-yl)-pentan-3-ols im Vergleich mit der bekannten Verbindung (A).

Beispiel F

Sproßbehandlungs-Test / Getreidemehltau / Protektiv
(blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator (Alkyl-aryl-polyglykolether) auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var. hordei.

Nach 6 Tagen Verweilzeit der Pflanzen bei einer Temperatur von 21 – 22° C und einer Luftfeuchtigkeit von 80 – 90% wertet man den Besatz der Pflanzen mit Mehltaupusteln aus. Der Befallsgrad wird in Prozent des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist um so wirksamer, je geringer der Mehltaubefall ist.

Wirkstoffe, Wirkstoffkonzentrationen in der Spritzbrühe und Befallsgrade gehen hervor aus der nachfolgenden Tabelle.

Tabelle F

Sproßbehandlungs-Test/Getreidemehltau/Protektiv

| Wirkstoffe | Wirkstoffkonzentration in der Spritzbrühe in Gew.-% | Befall in % der unbehandelten Kontrolle |
|---|---|---|
| 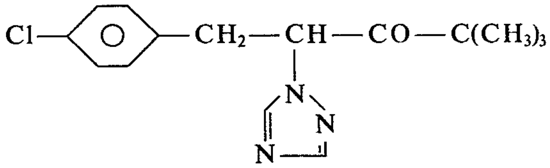 (A) (bekannt) | 0,001 | 72,5 |

Fortsetzung

| Wirkstoffe | Wirkstoff-konzentration in der Spritz-brühe in Gew.-% | Befall in % der unbehandelten Kontrolle |
|---|---|---|
| 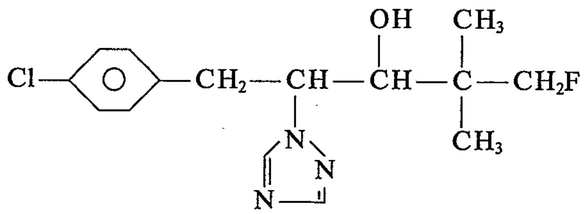 | 0,001 | 0,0 |

**Patentansprüche**

1. Substituierte Triazolylmethyl-tert.-butyl-ketone der Formel

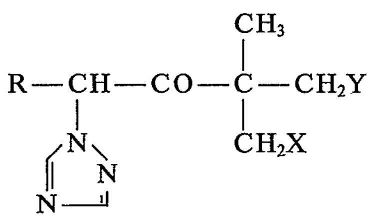

(I)

in welcher

R für Alkyl mit 1 bis 12 Kohlenstoffatomen, Alkenyl mit 2 bis 12 Kohlenstoffatomen, Alkinyl mit 2 bis 12 Kohlenstoffatomen, gegebenenfalls durch Alkyl mit 1 bis 4 Kohlenstoffatomen substituiertes Cycloalkyl mit 3 bis 7 Kohlenstoffatomen, gegebenenfalls durch Alkyl mit 1 bis 4 Kohlenstoffatomen substituiertes Cycloalkylalkyl mit 3 bis 7 Kohlenstoffatomen im Cycloalkylteil und 1 bis 4 Kohlenstoffatomen im Alkylteil sowie für Aralkyl mit 6 bis 10 Kohlenstoffatomen im Arylteil und 1 bis 4 Kohlenstoffatomen im Alkylteil steht, wobei der Arylteil substituiert sein kann durch Alkyl mit 1 bis 4 Kohlenstoffatomen, Alkoxy mit 1 bis 4 Kohlenstoffatomen, Alkylthio mit 1 bis 4 Kohlenstoffatomen, Halogen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Halogenalkylthio und 1 bis 4 Kohlenstoffatomen und 1 bis 5 gleichen oder verschiedenen Halogenatomen, Nitro, Cyano, Alkoxycarbonyl mit 1 bis 4 Kohlenstoffatomen im Alkoxyteil, Amino, Alkylamino mit 1 bis 4 Kohlenstoffatomen, Dialkylamino mit 1 bis 4 Kohlenstoffatomen in jedem Alkylteil, Phenylamino, gegebenenfalls durch Halogen oder Alkyl mit 1 oder 2 Kohlenstoffatomen substituiertes Phenoxy und/oder gegebenenfalls durch Halogen oder Alkyl mit 1 oder 2 Kohlenstoffatomen substituiertes Phenyl,

X für Wasserstoff steht und

Y für Fluor oder Chlor steht oder

X und Y gleich sind und für Fluor oder Chlor stehen,

sowie deren pflanzenverträgliche Säureadditions-Salze und Metallsalz-Komplexe.

2. Verfahren zur Herstellung von substituierten Triazolylmethyl-tert.-butyl-ketonen der Formel

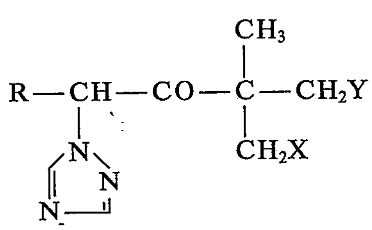

(I)

in welcher

R für Alkyl mit 1 bis 12 Kohlenstoffatomen, Alkenyl mit 2 bis 12 Kohlenstoffatomen, Alkinyl mit 2 bis 12 Kohlenstoffatomen, gegebenenfalls durch Alkyl mit 1 bis 4 Kohlenstoffatomen substituiertes Cycloalkyl mit 3 bis 7 Kohlenstoffatomen, gegebenenfalls durch Alkyl mit 1 bis 4 Kohlenstoffatoamen substituiertes Cycloalkylalkyl mit 3 bis 7 Kohlenstoffatomen im Cycloalkylteil und 1 bis 4 Kohlenstoffatomen im Alkylteil sowie für Aralkyl mit 6 bis 10 Kohlenstoffatomen im Arylteil und 1 bis 4 Kohlenstoffatomen im Alkylteil steht, wobei der Arylteil substituiert sein kann durch Alkyl mit 1 bis 4 Kohlenstoffatomen, Alkoxy mit 1 bis 4 Kohlenstoffatomen, Alkylthio mit 1 bis 4 Kohlenstoffatomen, Halogen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Halogenalkylthio und 1 bis 4 Kohlenstoffatomen und 1 bis 5 gleichen oder verschiedenen Halogenatomen, Nitro, Cyano, Alkoxycarbonyl mit 1 bis 4 Kohlenstoffatomen im Alkoxyteil, Amino, Alkylamino mit 1 bis 4 Kohlenstoffatomen, Dialkylamino mit 1 bis 4 Kohlenstoffatomen in jedem Alkylteil, Phenylamino, gegebenenfalls durch Halogen oder Alkyl mit 1 oder 2 Kohlenstoffatomen substituiertes Phenoxy und/oder gegebenenfalls durch Halogen oder Alkyl mit 1 oder 2 Kohlenstoffatomen substituiertes Phenyl,

X für Wasserstoff steht und

Y für Fluor oder Chlor steht oder

X und Y gleich sind und für Fluor oder Chlor stehen,

sowie von deren pflanzenverträglichen Säureadditions-Salzen und Metallsalz-Komplexen, dadurch gekennzeichnet, daß man Triazolylmethyl-tert.-butyl-ketone der Formel

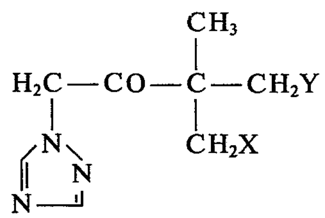

(II)

in welcher

X und Y die oben angegebene Bedeutung haben,

mit einem Alkylierungsmittel der Formel

$$R-Z \qquad (III)$$

in welcher

R die oben angegebene Bedeutung hat und

Z für eine elektronenanziehende Abgangsgruppierung steht,

in Gegenwart einer Base und in Gegenwart eines organischen Verdünnungsmittels oder in einem wäßrig-organischen Zweiphasensystem in Gegenwart eines Phasentransferkatalysators umsetzt und gegebenenfalls anschließend eine Säure oder ein Metallsalz addiert.

3. Pflanzenschutzmittel, gekennzeichnet durch einen Gehalt an mindestens einem substituierten Triazolylmethyl-tert.-butyl-keton der Formel (I) bzw. einem Säureadditions-Salz oder Metallsalz-Komplex eines Stoffes der Formel (I).

4. Verfahren zur Bekämpfung von Pilzen, dadurch gekennzeichnet, daß man substituierte Triazolylmethyl-tert.-butyl-ketone der Formel (I) bzw. Säureadditions-Salze oder Metallsalz-Komplexe von Stoffen der Formel (I) auf Pilze oder ihren Lebensraum einwirken läßt.

5. Verfahren zur Regulierung des Pflanzenwachstums, dadurch gekennzeichnet, daß man substituierte Triazolylmethyl-tert.-butyl-ketone der Formel (I) bzw. Säureadditions-Salze oder Metallsalz-Komplexe von Stoffen der Formel (I) auf die zu behandelnden Pflanzen oder ihren Lebensraum einwirken läßt.

6. Verwendung von substituierten Triazolylmethyl-tert.-butyl-ketonen der Formel (I) bzw. von Säureadditions-Salzen oder Metallsalz-Komplexen von Stoffen der Formel (I) als Pflanzenschutzmittel.

7. Verwendung von substituierten Triazolylmethyl-tert.-butyl-ketonen der Formel (I) bzw. von Säureadditions-Salzen oder Metallsalz-Komplexen von Stoffen der Formel (I) zur Bekämpfung von Pilzen.

8. Verwendung von substituierten Triazolylmethyl-tert.-butyl-ketonen der Formel (I) bzw. von Säureadditions-Salzen oder Metallsalz-Komplexen von Stoffen der Formel (I) zur Regulierung des Pflanzenwachstums.

9. Verwendung von substituierten Triazolylmethyl-tert.-butyl-ketonen der Formel (I) bzw. von Säureadditions-Salzen oder Metallsalz-Komplexen von Stoffen der Formel (I) als Zwischenprodukte zur Herstellung von Pflanzenschutzmitteln.

10. Verfahren zur Herstellung von Pflanzenschutzmitteln, dadurch gekennzeichnet, daß man substituierte Triazolylmethyl-tert.-butyl-ketone der Formel (I) bzw. Säureadditions-Salze oder Metallsalz-Komplexe von Stoffen der Formel (I) mit Steckmitteln und/oder oberflächenaktiven Stoffen vermischt.

**Claims**

1. Substituted triazolylmethyl tert.-butyl ketones of the formula

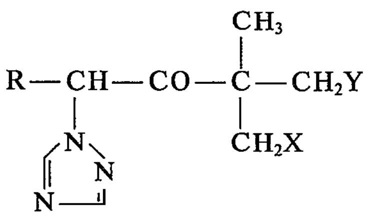

(I)

in which

R represents alkyl with 1 to 12 carbon atoms, alkenyl with 2 to 12 carbon atoms, alkinyl with 2 to 12 carbon atoms, cycloalkyl which has 3 to 7 carbon atoms and is optionally substituted by alkyl with 1 to 4 carbon atoms, cycloalkylalkyl which has 3 to 7 carbon atoms in the cycloalkyl part and 1 to 4 carbon atoms in the alkyl part and is optionally substituted by alkyl with 1 to 4 carbon atoms, as well as aralkyl with 6 to 10 carbon atoms in the aryl part and 1 to 4 carbon atoms in the alkyl part, it being possible for the aryl part to be substituted by alkyl with 1 to 4 carbon atoms, alkoxy with 1 to 4 carbon atoms, alkylthio with 1 to 4 carbon atoms, halogen, halogenoalkyl with 1 to 4 carbon atoms and 1 to 5 identical or different halogen atoms, halogenoalkoxy with 1 to 4 carbon atoms and 1 to 5 identical or different halogen atoms, halogenoalkylthio and 1 to 4 carbon atoms and 1 to 5 identical or different halogen atoms, nitro, cyano, alkoxycarbonyl with 1 to 4 carbon atoms in the alkoxy part, amino, alkylamino with 1 to 4 carbon atoms, dialkylamino with 1 to 4 carbon atoms in each alkyl part, pehenylamino, phenoxy which is optionally substituted by halogen or alkyl with 1 or 2 carbon atoms and/or phenyl which is optionally substituted by halogen or alkyl with 1 or 2 carbon atoms,

X represents hydrogen and

Y represents fluorine or chlorine or

X and Y are identical and represent fluorine or chlorine,

and acid addition salts and metal salt complexes thereof which are tolerated by plants.

2. Process for the preparation of substituted triazolylmethyl tert.-butyl ketones of the formula

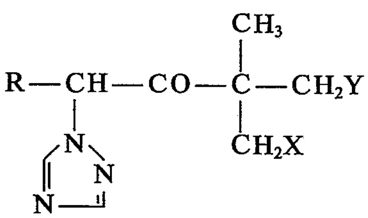

(I)

in which

R represents alkyl with 1 to 12 carbon atoms, alkenyl with 2 to 12 carbon atoms, alkinyl with 2 to 12 carbon atoms, cycloalkyl which has 3 to 7 carbon atoms and is optionally substituted by alkyl with 1 to 4 carbon atoms, cycloalkylalkyl which has 3 to 7 carbon atoms in the cycloalkyl part and 1 to 4 carbon atoms in the alkyl part and is optionally substituted by alkyl with 1 to 4 carbon atoms, as well as aralkyl with 6 to 10 carbon atoms in the aryl part and 1 to 4 carbon atoms in the alkyl part, it being possible for the aryl part to be substituted by alkyl with 1 to 4 carbon atoms, alkoxy with 1 to 4 carbon atoms, alkylthio with 1 to 4 carbon atoms, halogen, halogenoalkyl with 1 to 4 carbon atoms and 1 to 5 identical or different halogen atoms, halogenoalkoxy with 1 to 4 carbon atoms and 1 to 5 identical or different halogen atoms, halogenoalkylthio and 1 to 4 carbon atoms and 1 to 5 identical or different halogen atoms,

nitro, cyano, alkoxycarbonyl with 1 to 4 carbon atoms in the alkoxy part, amino, alkylamino with 1 to 4 carbon atoms, dialkylamino with 1 to 4 carbon atoms in each alkyl part, phenylamino, phenoxy which is optionally substituted by halogen or alkyl with 1 or 2 carbon atoms and/or phenyl which is optionally substituted by halogen or alkyl with 1 or 2 carbon atoms,

X represents hydrogen and
Y represents fluorine or chlorine or
X and Y are identical and represent fluorine or chlorine,

and acid addition salts and metal salt complexes thereof which are tolerated by plants, characterised in that triazolylmethyl tert.-butyl ketones of the formula

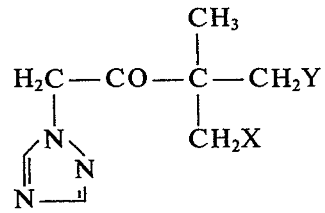

(II)

in which

X and Y have the meaning indicated above,
are reacted with an alkylating agent of the formula

$$R-Z \qquad (III)$$

in which

R has the meaning indicated above and
Z represents an electron-attracting leaving group,

in the presence of a base and in the presence of an organic diluent, or in an aqueous-organic two-phase system in the presence of a phase transfer catalyst, and if required, an acid or a metal salt is then added on.

3. Plant protection agent, characterised in that it contains at least one substituted triazolylmethyl tert.-butyl ketone of the formula(I) or an acid addition salt or metal salt complex of a compound of the formula (I).

4. A method of combating fungi, characterised in that substituted triazolylmethyl tert.-butyl ketones of the formula (I) or acid addition salts or metal salt complexes of compounds of the formula (I) are allowed to act on fungi or their habitat.

5. A method of regulating plant growth, characterised in that substituted triazolylmethyl tert.-butyl ketones of the formula (I) or acid addition salts or metal salt complexes of compounds of the formula (I) are allowed to act on the plants to be treated or their habitat.

6. Use of substituted triazolylmethyl tert.-butyl ketones of the formula (I) or of acid addition salts or metal salt complexes of compounds of the formula (I) as plant protection agents.

7. Use of substituted triazolylmethyl tert.-butyl ketones of the formula (I) or of acid addition salts or metal salt complexes of compounds of the formula (I) for combating fungi.

8. Use of substituted triazolylmethyl tert.-butyl ketones of the formula (I) or of acid addition salts or metal salt complexes of compounds of the formula (I) for regulating plant growth.

9. Use of substituted triazolylmethyl tert.-butyl ketones of the formula (I) or of acid addition salts or metal salt complexes of compounds of the formula (I) as intermediate products for the preparation of plant protection agents.

10. Process for the preparation of plant protection agents, characterised in that substituted triazolylmethyl tert.-butyl ketones of the formula (I) or acid addition salts or metal salt complexes of compounds of the formula (I) are mixed with extenders and/or surface-active compounds.

**Revendications**

1. Triazolylméthyl-tert.-butyl-cétones substituées de formule:

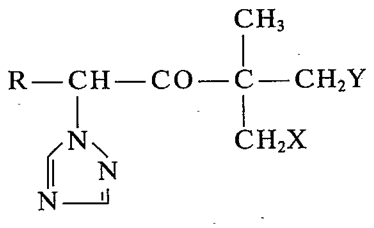

(I)

dans laquelle

R représente un groupe alkyle contenant 1 à 12 atomes de carbone, un groupe alcényle contenant 2 à 12 atomes de carbone, un groupe alcinyle contenant 2 à 12 atomes de carbone, un groupe cycloalkyle contenant 3 à 7 atomes de carbone et éventuellement substitué par un groupe alkyle contenant 1 à 4 atomes de carbone, un groupe cycloalkylalkyle contenant 3 à 7 atomes de carbone dans la fraction cycloalkyle et 1 à 4 atomes de carbone dans la fraction alkyle et éventuellement substitué par un groupe alkyle contenant 1 à 4 atomes de carbone, de même qu'un groupe aralkyle contenant 6 à 10 atomes de carbone dans la fraction aryle et 1 à 4 atomes de carbone dans la fraction alkyle, la fraction aryle pouvant être substituée par un groupe alkyle contenant 1 à 4 atomes de carbone, par un groupe alcoxy contenant 1 à 4 atomes de carbone, par un groupe alkylthio contenant 1 à 4 atomes de carbone, par un atome d'halogène, par un groupe halogénalkyle contenant 1 à 4 atomes de carbone et 1 à 5 atomes d'halogènes identiques ou différents, par un groupe halogénalcoxy contenant 1 à 4 atomes de carbone et 1 à 5 atomes d'halogènes identiques ou différents, par un groupe halogénalkylthio contenant 1 à 4 atomes de carbone et 1 à 5 atomes d'halogènes identiques ou différents, par un groupe nitro, par un groupe cyano, par un groupe alcoxycarbonyle contenant 1 à 4 atomes de carbone dans la fraction alcoxy, par un groupe amino, par un groupe alkylamino contenant 1 à 4 atomes de carbone, par un groupe dialkylamino contenant 1 à 4 atomes de carbone dans chaque fraction alkyle, par un groupe phénylamino, par un groupe phénoxy éventuellement substitué par un atome d'halogène ou par un groupe alkyle contenant 1 ou 2 atomes de carbone et/ou par un groupe phényle éventuellement substitué par un atome d'halogène ou par un groupe alkyle contenant 1 ou 2 atomes de carbone,

X représente un atome d'hydrogène et

Y représente un atome de fluor ou de chlore, ou

X et Y sont identiques et représentent chacun un atome de fluor ou de chlore,

de même que leurs complexes de sels métalliques et leurs sels d'addition d'acide compatibles avec les plantes.

2. Procédé de préparation de triazolylméthyl-tert.-butyl-cétones substituées de formule:

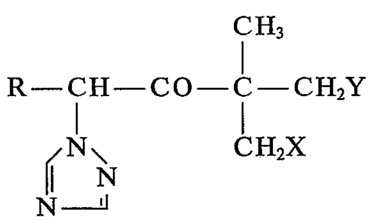

(I)

dans laquelle

R représente un groupe alkyle contenant 1 à 12 atomes de carbone, un groupe alcényle contenant 2 à 12 atomes de carbone, un groupe cycloalkyle contenant 3 à 7 atomes de carbone et éventuellement substitué par un groupe alkyle contenant 1 à 4 atomes de carbone, un groupe cycloalkylalkyle contenant 3 à 7 atomes de carbone dans la fraction cycloalkyle et 1 à 4 atomes de carbone dans la fraction alkyle et éventuellement substitué par un groupe alkyle contenant 1 à 4 atomes de carbone, un groupe aralkyle contenant 6 à 10 atomes de carbone dans la fraction aryle et 1 à 4 atomes de carbone dans la fraction alkyle, la fraction aryle pouvant être substituée par un groupe alkyle contenant 1 à 4 atomes de carbone, par un groupe alcoxy contenant 1 à 4 atomes de carbone, par un groupe alkylthio contenant 1 à 4 atomes de carbone, par un atome d'halogène, par un groupe halogénalkyle contenant 1 à 4 atomes de carbone et 1 à 5 atomes d'halogènes identiques ou différents, par un groupe halogénalcoxy contenant 1 à 4 atomes de carbone et 1 à 5 atomes d'halogènes identiques ou

différents, par un groupe halogénalkylthio contenant 1 à 4 atomes de carbone et 1 à 5 atomes d'halogènes identiques ou différents, par un groupe nitro, par un groupe cyano, par un groupe alcoxycarbonyle contenant 1 à 4 atomes de carbone dans la fraction alcoxy, par un groupe amino, par un groupe alkylamino contenant 1 à 4 atomes de carbone, par un groupe dialkylamino contenant 1 à 4 atomes de carbone dans chaque fraction alkyle, par un groupe phénylamino, par un groupe phénoxy éventuellement substitué par un atome d'halogène ou par un groupe alkyle contenant 1 ou 2 atomes de carbone et/ou par un groupe phényle éventuellement substitué par un atome d'halogène ou par un groupe alkyle contenant 1 ou 2 atomes de carbone,

X représente un atome d'hydrogène, et
Y représente un atome de fluor ou de chlore ou
X et Y sont identiques et représentent chacun un atome de fluor ou de chlore,

ainsi que de leurs complexes de sels métalliques et de leurs sels d'addition d'acide compatibles avec les plantes, caractérisé en ce qu'on fait réagir des triazolylméthyl-tert.-butyl-cétones de formule:

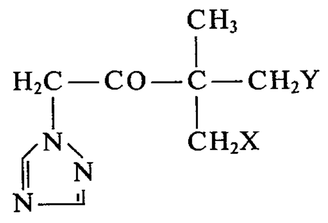

(II)

dans laquelle

X et Y ont les significations indiquées ci-dessus,

avec un agent d'alkylation de formule:

$$R-Z \quad (III)$$

dans laquelle

R a la signification indiquée ci-dessus et
Z représente un groupement qui s'éloigne et qui attire les électrons,

en présence d'une base, ainsi qu'en présence d'un diluant organique, ou dans un système à deux phases (aqueux-organique) en présence d'un catalyseur de transfert de phases, puis on ajoute éventuellement un acide ou un sel métallique.

3. Agent de protection des plantes, caractérisé en ce qu'il contient au moins une triazolylméthyl-tert.-butyl-cétone substituée de formule (I) ou un sel d'addition d'acide ou encore un complexe de sel métallique d'une substance de formule (I).

4. Procédé en vue de combattre les champignons, caractérisé en ce qu'on laisse agir des triazolylméthyl-tert.-butyl-cétones substituées de formule (I) ou des sels d'addition d'acide ou encore des complexes de sels métalliques de substances de formule (I) sur des champignons ou leur biotope.

5. Procédé en vue de régler la croissance des plantes, caractérisé en ce qu'on laisse agir des triazolylméthyl-tert.-butyl-cétones substituées de formule (I) ou des sels d'addition d'acide ou encore des complexes de sels métalliques de substances de formule (I) sur les plantes à traiter ou leur biotope.

6. Utilisation de triazolylméthyl-tert.-butyl-cétones substituées de formule (I) ou de sels d'addition d'acide ou encore de complexes de sels métalliques de substances de formule (I) comme agents de protection des plantes.

7. Utilisation de triazolylméthyl-tert.-butyl-cétones substituées de formule (I) ou de sels d'addition d'acide ou encore de complexes de sels métalliques de substances de formule (I) pour combattre les champignons.

8. Utilisation de triazolylméthyl-tert.-butyl-cétones substituées de formule (I) ou de sels d'addition d'acide ou encore de complexes de sels métalliques de substances de formule (I) pour régler la croissance des plantes.

9. Utilisation de triazolylméthyl-tert.-butyl-cétones substituées de formule (I) ou de sels d'addition d'acide ou encore de complexes de sels métalliques de substances de formule (I) comme produits intermédiaires pour la préparation d'agents de protection des plantes.

10. Procédé de préparation d'agents de protection des plantes, caractérisé en ce qu'on mélange des triazolylméthyl-tert.-butyl-cétones substituées de formule (I) ou des sels d'addition d'acide ou encore des complexes de sels métalliques de substances de formule (I) avec des diluants et/ou des substances tensio-actives.